Europäisches Patentamt

European Patent Office

Office européen des brevets

(11) Publication number: **0 422 253 A1**

# EUROPEAN PATENT APPLICATION

(21) Application number: **89118682.7**

(22) Date of filing: **07.10.89**

(51) Int. Cl.5: **A61M 21/00, A61B 5/00**

(43) Date of publication of application:
**17.04.91 Bulletin 91/16**

(84) Designated Contracting States:
**AT BE CH DE FR GB IT LI LU NL SE**

(71) Applicant: **PROFIT TECHNOLOGY INCORPORATED**
**17 Battery Place- 14th Floor**
**New York, N.Y. 10004(US)**

(72) Inventor: **Bradford, Michael H.**
**22 Kilmar Drive**
**Manalapan New Jersey 07726(US)**

(74) Representative: **Hoeger, Stellrecht & Partner**
**Uhlandstrasse 14 c**
**W-7000 Stuttgart 1(DE)**

(54) **Field effect processor.**

(57) A system for monitoring and modifying the biophysical characteristics of a human user. A complete biofeedback loop is realized comprising a signal generating and processing device, a plurality of transducers (13), the human user (10) and a plurality of sensors (14). The transducers and sensors create a bioacoustical environment by utilizing audio, video, RF and biostatic signals.

A biophysical acceptance/rejectance profile of the human user is computed which provides information relating to the biophysical characteristics of the human user. The acceptance/rejectance profile of the human user is compared to a predetermined desired acceptance/rejectance profile in order to modify the transduced signals to bring the human user to a predetermined desired biophysical state.

FIG. 1

Xerox Copy Centre

## FIELD OF THE INVENTION

The present invention relates generally to the fields of biofeedback and stress reduction and more particularly to a system for monitoring and stimulating various biophysical signals of a human being. The present invention can be used for relaxation, psychotherapy, superlearning, education, creativity, problem solving and idea generation.

## BACKGROUND OF THE INVENTION

In the modern world filled with all of its complexities, it is often difficult for an individual to find a release from stress and tension. Such a release from the pressures of daily life is important for education, creativity, problem solving and idea generation. The fields of medicine and psychology are beginning to realize the detrimental effects of the constant pressures and stress associated with modern day living, and the importance of finding methods for relaxation and reducing stress and tension. Thus, there is shown the need for assisting individuals with their problems of dealing with stress and pressure.

Modern psychotherapy stress reduction techniques typically require both a patient and a therapist. The patient usually engages in relaxed conversation or some type of related activity while the therapist observes the patient's reaction to various stages of the activity. In the field of psychology, for example, a psychologist attempts to solve the fears and problems of a patient in order to reduce various conflicts and stress in the patient's life which may be created by these problems and fears. A psychologist typically has a patient rest comfortably on a couch or chair and engages in conversation with the psychologist. The patient attempts to express various problems and fears to the psychologist, which are then analyzed by the psychologist in order to suggest solutions to the patient. This is a long and involved process which requires the rather inconvenient and expensive presence of the psychologist. Many people who seek the services of a psychologist are disappointed because they are unable to express themselves in the presence of another and cannot overcome their apprehension of having their fears and problems known by the psychologist.

Hypnosis is another stress reduction or relaxation technique which requires both a patient and a therapist. The therapist is a hypnotist who attempts to induce the patient into a certain relaxed state called a hypnotic trance. In this state the patient, with the help of the experienced hypnotist, tries to express and find solutions for various fears and problems from which the patient may suffer. This technique is also very expensive and finds limited success since the hypnotist cannot always gain the confidence of the patient as required to induce a hypnotic trance.

Another reason for the limited success of these psychotherapy techniques lies in the subjective nature by which the therapist must analyze the various reactions of the patient. It may take a very long time for a therapist to get to know the patient before the therapist may even begin attempting to understand the often times complex problems and fears of a particular patient.

Modern biofeedback practices typically consist of techniques which merely monitor the biophysical characteristics of an individual. The results of these measurements are then made available to the monitored individual, such as by a numeric readout representing an individual's heart rate. Such modern biofeedback techniques do not use the monitored results to modify or stimulate the individual to produce a desired reaction which would alter these monitored results so as to bring the individual to a predetermined desired condition. Thus, modern biofeedback techniques are not true feedback techniques since the essential feedback loop is severed after the monitored results are gathered.

Furthermore, the effectiveness of such modern biofeedback techniques is usually diminished since monitoring devices are physically attached to the monitored individual, thereby interfering with his ability to concentrate on his biophysical characteristics.

Thus, the need is shown for a stress reduction or relaxation technique which may be used by a single individual without the requirement of a therapist being present. Preferably such a technique would utilize the biophysical characteristics of an individual without the use of distracting monitoring devices. Additionally, the biophysical characteristics should be used to stimulate the individual in order to help bring the individual to a predetermined desired biophysical condition. Such a technique would eliminate an individual's apprehension of disclosing thoughts, feelings, fears and problems to another person and avoid the inconvenience and expense of an experienced therapist.

## SUMMARY OF THE INVENTION

It is a primary object of the present invention to provide a system for producing various transduced signals which stimulate and modify the biophysical characteristics of an individual in response to the biophysical state and biophysical changes of the individual. It is a related object of the invention to produce such transduced signals which selectively stimulate particular biophysical characteristics of an individual.

Another object of the present invention is to provide such a system which teaches or trains an individual to be aware of and control their various biophysical characteristics. In this regard, a related object of the invention is to teach an individual to utilize their awareness and control of their biophysical characteristics to relax negative muscle tensions.

Another object of the invention is to provide such a system which creates an environment conducive to training an individual to be aware of and control their various biophysical characteristics.

Still another object of the invention is to provide such a system for creating an environment in which a user is particularly responsive to changes in their biophysical characteristics.

Yet another object of the present invention is to provide such a system for monitoring and modifying the biophysical characteristics of an individual without the use of any physically attached monitoring devices.

The above objects are realized in accordance with the system of the present invention which provides a controlled bioacoustic environment for an individual comprising a plurality of transducers and sensors for monitoring and affecting, respectively, a plurality of biophysical characteristics of the individual. The system continually compares the signals sent by the transducers to the signals received by the sensors, thereby determining an acceptance/rejectance profile of the individual. This acceptance/rejectance profile represents the biophysical signals the individual is accepting and the biophysical signals the individual is rejecting. Using this acceptance/rejectance profile, possibly also in combination with a predetermined desired acceptance/rejectance profile, the system modifies the signals sent to the transducers so as to bring the individual to a predetermined desired biophysical condition.

Every human being has many biophysical characteristics which make up the individual's particular biophysical condition. For example, body temperature, heartbeat, brainwave activity and respiration are all biophysical characteristics which are part of an individual's overall biophysical state. In addition to these well known biophysical characteristics, there are many biophysical characteristics of an individual which may be determined by the static, or when analyzed from a biophysical standpoint, the biostatic field generated by a human being. Properties of this biostatic field include intensity, polarity and frequency. The biostatic field has properties that are unique to the individual as well as properties that are unique to the species and gender. For example, men and women have properties unique to the individual, properties that are unique to their particular gender, and properties that are unique to them as individual human beings. It is an important aspect of the present invention to utilize this biostatic field to determine various biophysical characteristics and properties of an individual using the system.

According to a preferred embodiment of the invention, an individual lies comfortably on a table suspended within an aesthetically pleasing framework. Attached to the framework are several audio speakers which are positioned so the individual can hear audio signals generated by the system. A number of transducers, such as electromechanical vibrators, RF signal generators and colored lights are also provided for additionally affecting a user so as to invoke a particular desired biophysical response. A plurality of receiving devices such as microphones, RF receivers and electromagnetic receivers are provided which monitor the biophysical characteristics of the individual. An additional receiving device monitors the biostatic field of the user which provides information relating to the mental, physical and emotional state of the user.

These and other features and advantages of the present invention will be more readily apparent upon reading the following description of a preferred exemplified embodiment of the invention and upon reference to the accompanying drawings wherein:

BRIEF DESCRIPTION OF THE DRAWINGS

FIGURE 1 is a general flow diagram of the present invention;

FIG. 2 is a perspective view of a preferred embodiment of the invention;

FIG. 3 is a bottom view of the table used in conjunction with the present invention depicting a preferred configuration for biostatic foil sensors;

FIG. 4 is a bottom view of the table used in conjunction with the present invention depicting a preferred configuration for biostatic foil sensors and biostatic foam sensors;

FIG. 5 is a block diagram depicting the major functional elements of the present invention;

FIG. 6 is a block diagram depicting the functional elements of the field effect processor in accordance with the present invention;

FIGS. 7A-7D depict various embodiments of static conductive foam biostatic sensors for use in accordance with an important aspect of the present invention;

FIGS. 8A-8B depict various embodiments of static conductive gel biostatic sensors for use in accordance with an important aspect of the present invention.

While the invention will be described and disclosed in connection with certain preferred embodiments and procedures, it is not intended to limit the invention to those specific embodiments. Rather, it is intended to cover all such alternative embodiments and modifications as fall within the spirit and scope of the invention as defined by the appended claims.


## DETAILED DESCRIPTION OF THE PREFERRED EMBODIMENTS


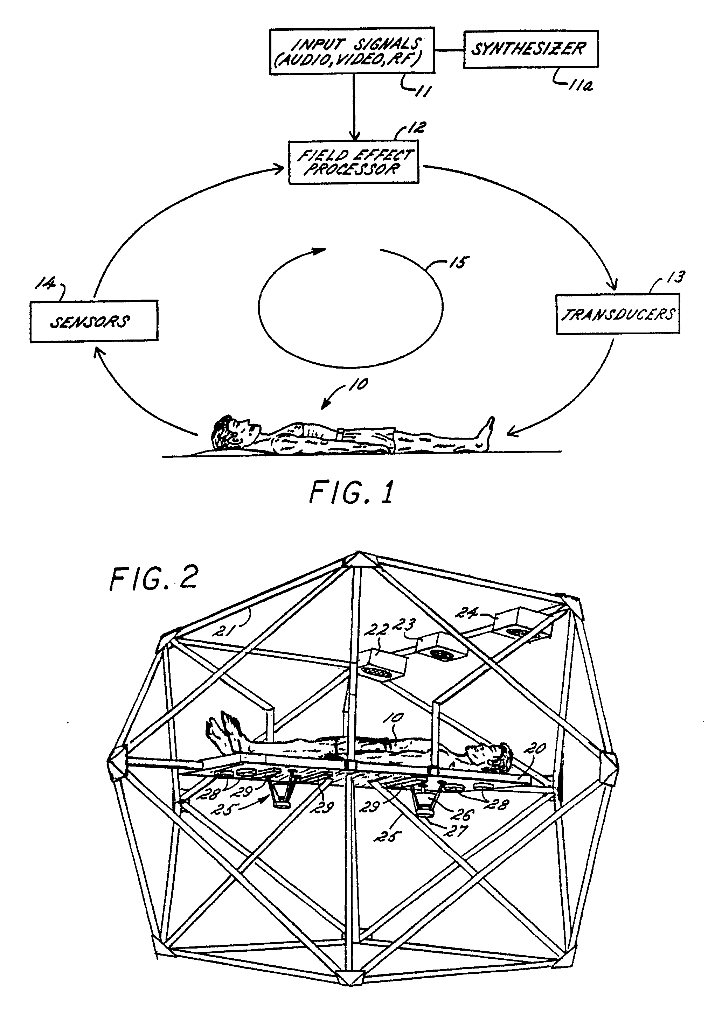

Turning now to the drawings and referring first to FIG. 1, there is shown a general flow diagram of the system in accordance with a preferred embodiment of the present invention. As can be seen, a human user 10 rests comfortably on a table, mattress or in some other relaxed position. The system is initially driven by using input signals 11 such as audio, video or RF signals. Preferably, the input signals 11 will be include audio signals in the form of musical recording as from tapes, phonographs or compact disks. Alternatively, or also in combination with such stored signals, the input signals 11 may include synthesized signals from a synthesizer 11a. The input signals 11 are supplied to a field effect processor 12 which initially supplies the input signals directly to the transducers 13. The transducers 13 convert the signals supplied by the field effect processor 12 into signals which may be perceived by the human user 10. Typically, the transduced signals will take the form of audio, video or RF signals or any combination thereof, and may include any signals which may be perceived consciously or subconsciously by the human user 10.

As can be seen in FIG. 1, the human user 10 is an integral part of the system. The human user 10 will react to the various signals produced by the transducers 13. Typically, a human user 10 will accept some of each of the transduced signals, while also rejecting some of each of the transduced signals. The accepted signals are absorbed in various areas of the body, indicating that the human user 10 is particularly responsive to this signal.

Since each of the transduced signals may be comprised of any number of complex signals, the body may be responsive only to certain portions of this complex signal. In such a case, the human user 10 will accept certain portions of the transduced signals to which he is responsive while he will reject certain portions of the transduced signals to which he is not responsive. Thus, an acceptance/rejectance profile of the user 10 may be obtained which provides information relating to the human user's 10 acceptance and rejection of the various signals sent by the transducers 13.

A plurality of sensors 14 are located within the vicinity of the human user 10 and receive signals which have not been absorbed by the human user 10. Similar to the signals produced by the transducers 13, these sensors 14 will receive signals in the form of audio, video, RF signals or other signals. Preferably, the sensors also include one or more biostatic sensors which provide information relating to the human user's 10 particular biostatic state. These signals received by the sensors 14 are supplied to the field effect processor 12.

The field effect processor 12 compares the signals it receives from the sensors 14 to the signals it supplies to the transducers 13. Using this comparison, the field effect processor 12 computes the acceptance/rejectance profile for the human user 10 for each of the biophysical signals being monitored by the system. Using this acceptance/rejectance profile, the field effect processor 12 modifies the supplied input signals 11 to alter the environment of the user 10 as created by the various transducers 13. The modification will create an environment which is more conducive to bringing the user 10 to a particular predetermined biophysical state. Thus, by using the signals received by the sensors 14, which contain information regarding the signals which have not been absorbed by the human user 10, to modify the signals produced by the the transducers 13, a true complete feedback loop 15 is realized.

According to an important aspect of the invention, the human user 10 is an integral element of the feedback system and provides information to the field effect processor 12 via the transducers 13 and the sensors 14 concerning the particular biophysical state of the human user 10. The field effect processor 12 uses this information from the feedback loop 15 to modify the signals it subsequently sends to the transducers 13. The field effect processor may be utilized in this configuration for the purpose of affecting and bringing the human user 10 to a predetermined desired biophysical state.

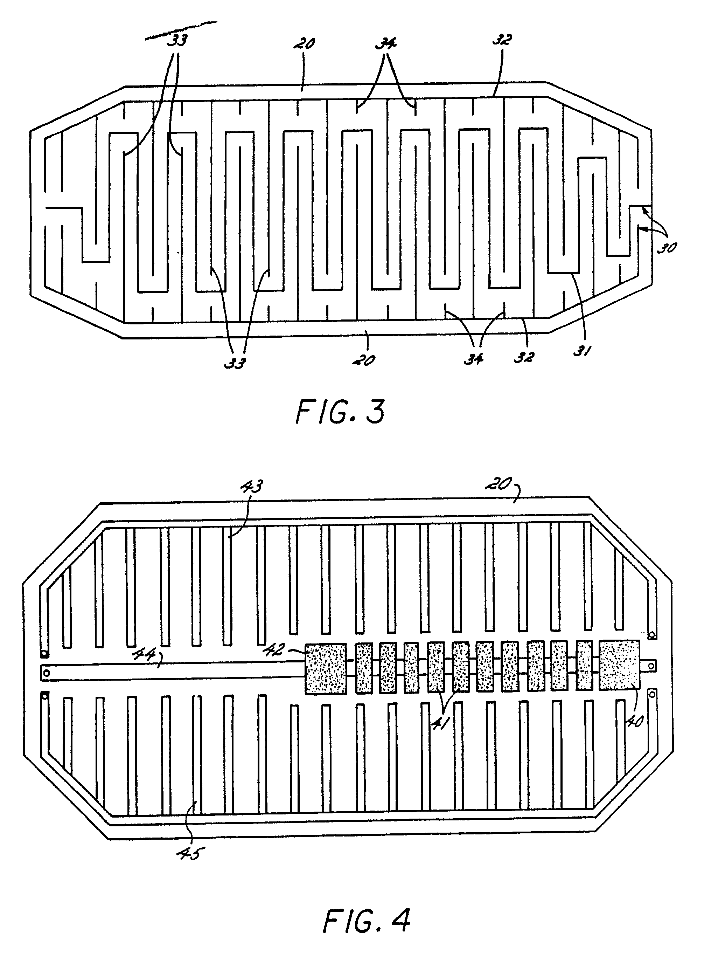

Turning next to Fig. 2, there is shown a perspective view of a preferred embodiment of the present invention. A table 20 is provided to support a human user 10 in a comfortable position. The table 20 is suspended within an aesthetically pleasing framework 21 and provides support for various transducers and sensors used by the system. A number of speakers 22, 23 and 24 are provided to output audio signals from the field affect processor to effect the human user 10. Preferably, the output of speakers 22, 24 and 23 correspond to the right, left and a triphonics signal, respectively, as from an input audio signal after modification by the field effect processor. The triphonics speaker 23, is supplied with the positive input leads from both the right 22 and the left 24 speakers, and thus, provides an audio signal which corresponds to the phase difference between the audio signals produced by the right 22 and left 24 speakers. This triphonics audio signal creates a particularly pleasing effect for the audio perception of the human user 10 and will help to relax the human user 10.

A number of audio vibrators 25 are attached to the bottom of the table 20 which transmit audio and subaudio vibrations to the human user 10 by using the table 20 as a sounding board. Preferably, the audio vibrators 25 will be comprised of both a speaker 26 and an electromechanical transducer 27 so as to effectively transmit the vibrations to the table 20 and the user 10. The electromechanical transducer 27 should operate in combination with the speaker 26 so as to initially excite the surface of the table 20 just prior to a signal being sent to activate or pulse the cone of the speaker 26. This excitation may be accomplished by energizing the electromechanical transducer 27 so as to create a small force by momentum transfer which sets the table 20 into slight motion, thereby overcoming the resistive tendencies of the table 20 with respect to sound waves generated by the speakers 26 which would be present if the table were to remain stationary. Since the electromechanical transducers 27 alleviate the resistive tendencies of the table 20, the sound waves produced by the speakers 26 are more effectively and more clearly transmitted to the table 20 and the human user 10. Thus the human user 10 will be able not only hear the audio signals from the speakers 22, 23 and 24, but will also be able to clearly feel audio and subaudio signals via the table 10 acting in combination with the speakers 26 of the vibrators 25.

Additional electromechanical vibrators 28 are provided and are attached directly to the underside of the table 20. Typically, these vibrators 28 will be energized at a relaxed heart or respiration rate. Alternately, they may be energized at some other biophysical harmonic rate as will be explained later. The electromechanical vibrators 28 may be grouped, such as a first group for the upper body portion and a second group for the lower body portion. In this case, these two groups may be alternately energized at a biophysically harmonic rate.

The system also comprises a plurality of biostatic sensors for sensing the biostatic field of the human user 10. The biostatic field is essentially the same as a conventional static field with the exception that the static field is created by the human user 10. Accordingly, the signals which the system senses relating to the biostatic field are analyzed and represent the various biostatic characteristics of the human user 10.

One of the preferred forms for the biostatic sensors is realized using static conductive foam 29 within the vicinity of the human user 10. Static conductive foam consists of a polyethylene or polyurethane sponge-like material which is impregnated with a conductive element such as carbon. One example of static conductive foam is Velostat™ which is manufactured by the 3M corporation. Conductive elements other than carbon may be impregnated in the foam such as barium titanate or silver chloride which may be particularly effective due to its hysteresis qualities.

The conductive foam 29 is preferably disposed on the surface or edges of the table. For example, the foam 29 may be disposed along the periphery of the table, and another group of static foam 29 may be positioned underneath the center of the table. The resistance of the static conductive foam 29 changes in relation to the biostatic field generated by the human user 10. By monitoring and utilizing the changes in the resistance of the foam 29 certain qualities of the biostatic field may be determined.

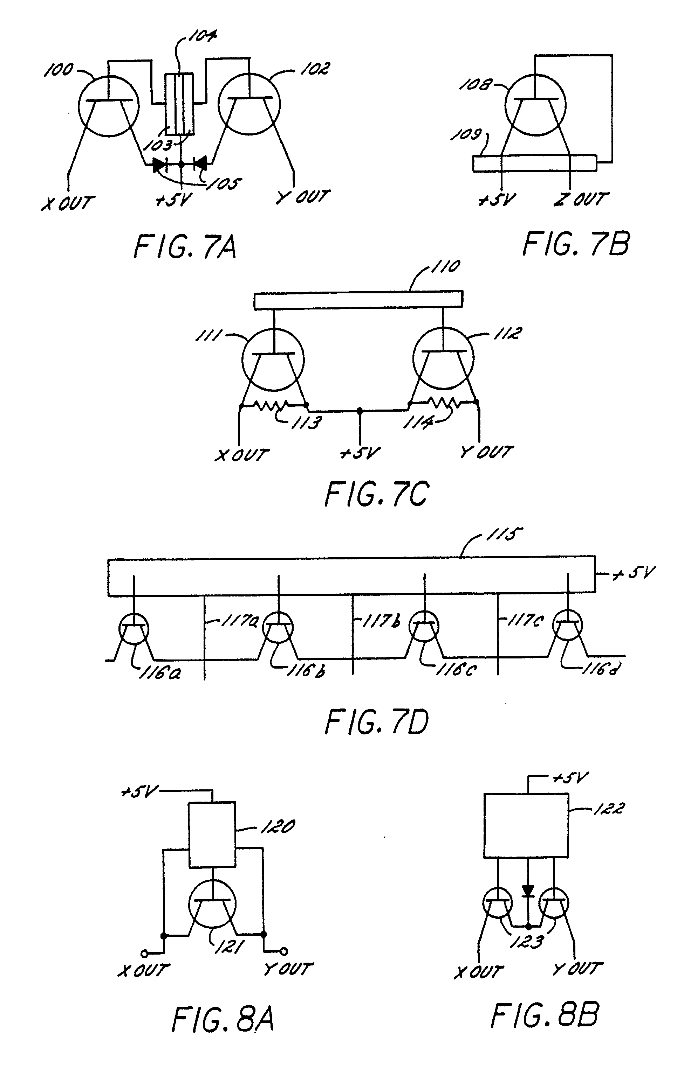

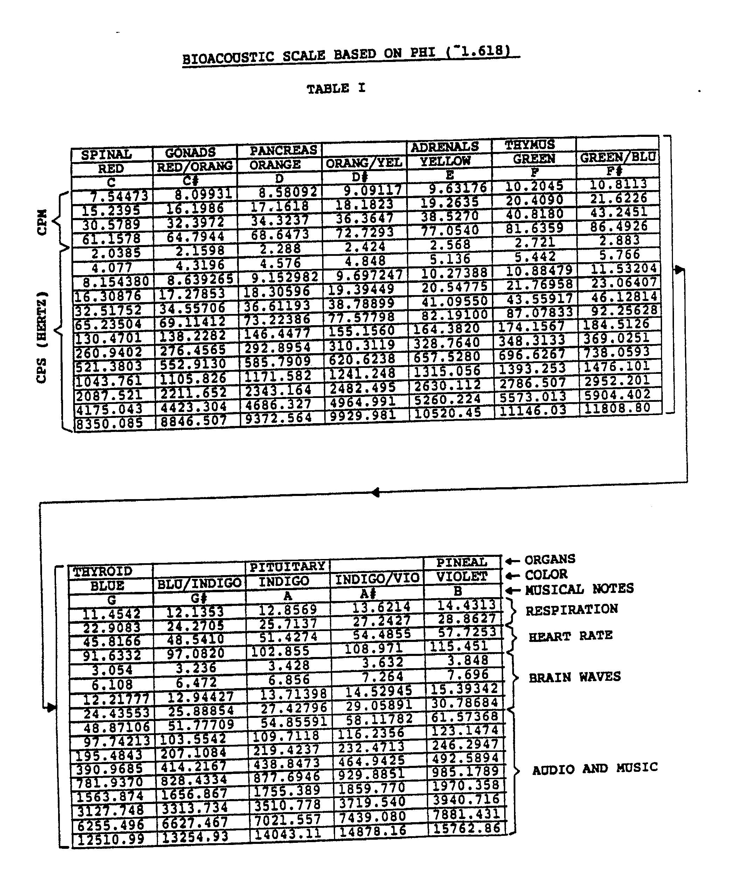

Turning for the moment to Figs. 7A-7D and Figs. 8A-88, there are shown several embodiments of biostatic sensors. The embodiments of Figs. 7A-7D are comprised of static conductive foam. In Fig. 7A the static conductive foam 103 is comprised of two sections with foil interposed between. The gates of two field effect transistors (FET's) 100, 102 are connected to either side of the foam 103, with their drain connected through diodes to a +5V source voltage running to the foil 104. The source leads of the FET's 100, 102 produce outputs $X_{out}$ and $Y_{out}$ which represent the biostatic field corresponding to the left and right (as shown) sides of the foam 103, respectively. Another configuration is depicted in Fig. 7B in which the source and drain leads of an FET 108 are run through a static conductive foam pad 109, while the gate is connected to one end of the foam pad 109. Either the drain or the source lead is connected to a +5V supply voltage, while the other lead produces an output voltage related to the biostatic field. A similar arrangement is shown in Fig. 7C which comprises 2 FET's 111, 112 with their gates connected to either end of a static conductive foam pad 110.

Between the FET's 111, 112 source and drain are connected resistors 113 and 114. Either the sources or the drains of the FET's 111, 112 are connected together to a +5V supply voltage, while the other leads $X_{out}$, $Y_{out}$ output the voltage corresponding to the biostatic field at either end of the conductive static foam pad 110. A similar configuration is presented in Fig. 7D which comprises a conductive foam pad 115 with a +5V supply voltage connected at one end. A plurality of FET's 116a, 116b, 116c and 116d are evenly spaced along the foam 115 with their drains attached to the foam 115. Their gates and drains are connected as shown and include intermediate leads 117a, 117b and 117c which are also connected to the source to drain connections at one end and to the foam 115 at the other end. The leads 117a, 117b and 117c output signals relating to the biostatic field at various positions along the static conductive foam pad 115.

Two more embodiments of biostatic sensors are shown in Figs. 8A and 8B, which comprise a non-conductive container, such as a plastic carton, filled with biostatic gel as the sensing means. The base of the gel is a silver chloride electrode gel which is combined with carbon, iron oxide and other conductive substances such as barium titanate to provide an effective biostatic indicator. As shown in Fig. 8A, one end of the container 120 holding static conductive gel is energized with a +5V supply voltage, while attached to the other end is the gate of an FET 120. The source and drain of the FET 120 are similarly inserted into the gel and also provide outputs $X_{out}$ and $Y_{out}$ related to the biostatic field at the two points where the source and drain leads are inserted into the gel.

The various embodiments of the biostatic sensors disclosed herein are disposed within the vicinity of the bioacoustic environment within which the human user is situated. The biostatic sensor devices may be advantageously disposed so as to provide desired biostatic field information. For example, the particular embodiment of Fig. 7D may be disposed transversely with respect to the table, thereby providing biostatic field information along a transverse axis of the human user. Alternatively, the sensor of Fig. 7D may be disposed longitudinally so as to provide biostatic field information of the human user on a longitudinal axis.

The signals provided to the field effect processor by the biostatic sensors are analyzed to determine polarity, density or amplitude and frequency of the biostatic field of the human user 10. The polarity of the biostatic signals relates to the human user's thought or reactions. In general, a negative thought or reaction will tend to produce a positive polarity of the biostatic field, a positive thought or reaction will tend to produce a negative polarity of the biostatic field, and a neutral or transitional thought or reaction will tend to produce a neutral polarity of the biostatic field. The density of the biostatic field relates to the human user's degree of relaxation. A higher density biostatic field indicates a user is relaxed, whereas a lower density biostatic field indicates a user is tense. The frequency of change of the biostatic field relates to the intensity of the user's reaction or emotions. A higher frequency of change indicates a high degree of emotion, whereas a lower frequency of change indicates a low degree of emotion.

Thus by analyzing this biostatic field, the system determines an emotional statement of the human user. This biostatic information is then utilized to modify the transduced signals affecting the human user to bring the human user to a predetermined desired biophysical state.

Another type of biostatic sensor may be realized using traces or mats of foil disposed along the surface of the table 20. A preferred configuration of the foil traces is depicted in Fig. 3 in which the foil traces 30 are disposed on the underside of the table 20. The foil traces 30 consist of two main sections according to this preferred configuration. One long trace of foil 31 is disposed in a serpentine fashion down the length of the table 20, and two separate traces 32 extend down along either edge of the table 20, each of which has long finger-like sections 33 extending into the serpentine trace 31 and short finger-like sections 34 extending up to the serpentine trace 31.

The right and left traces 32 along the edges of the table may be used to determine the difference in the biostatic field between the right and left portions of the human user. Similarly, the serpentine trace 31 as well as the traces 32 along the edges of the table 20 may be used to determine the difference in the biostatic field between the upper and lower portions of the human user.

According to another embodiment of the biostatic sensors as shown in Fig. 4, both biostatic conductive foam pads 41 and foil traces are used to sense the user's biostatic field. Two traces of foil 43 and 45 are provided on either side of the table 20. A number of static conductive foam pads 41 are disposed along the central longitudinal axis as well. Larger pads 40 and 42 are provided for the head and tailbone areas of the user respectively.

As can be seen from Figs. 3 and 4, the table 20 provided in the bioacoustical environment for the user to relax upon, actually functions as a comprehensive sensing device. The static conductive foam pads in combination with traces of foil provide a table which includes means for sensing the biostatic field of the user. Thus, the table functions not only as comfortable support for the user, but also as an antenna which senses the biostatic field of the user.

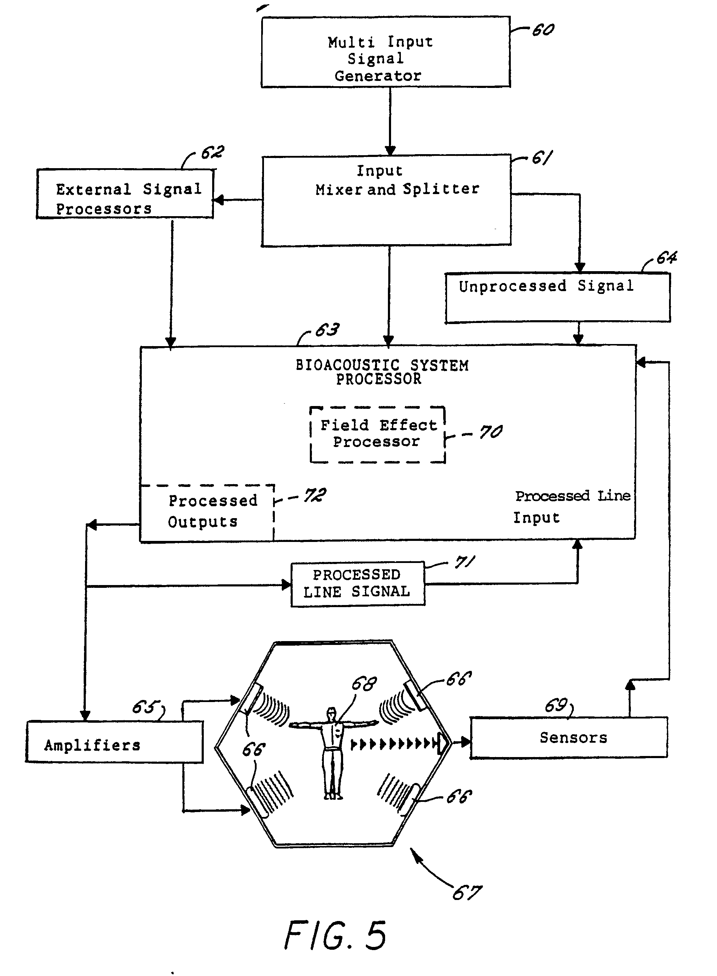

Turning now to Fig. 5, there is shown a functional flow diagram depicting the major elements of the system according to a preferred embodiment of the present invention. The system is supplied with predetermined input signals from a multi-input signal generator 60. Preferably, these signals may take the form of various musical recordings from conventional devices such as cassette tapes, compact disks or records. The multi-input signal generator 60 may also include various synthesizers or other nonmusical programmed signals. The predetermined input signals from the multi-input signal generator may additionally be comprised of video signals for stimulating the visual senses of the human user. The video signals may be displaying on a conventional display device to produce images of various shapes and colors.

A plurality of different predetermined input signals may be simultaneously provided to the system via the multi-input signal generator. In this regard, simultaneously input signals may be superimposed, such as several audio signals or several video signals, so as to combine the stimulating effects of each. The multi-input signal generator 60 supplies its various signals to a mixer and splitter 61 which manually or electronically mixes the signals from the multi-input signal generator 60 to predetermined desired levels and splits the signals for distribution and processing to external signal processors 62 and the bioacoustic system processor 63.

The external signal processors 62 operate to enhance the original signals from the multi-input signal generator 60 and provide more processing flexibility than that contained within the mixer and splitter 61. The enhancements will typically include various effects such as reverberation, tremolo, echo or other similar effects which are well known from the art of musical synthesizers. The external signal processors 62 may also include various time delays which may be set to compensate for various system delays or create a particularly desirable effect on the human user. The mixer and splitter 61 also provides an unprocessed signal 64 from the multi-input signal generator 60 to the bioacoustic system processor 63, which later utilizes this signal in a comparison process.

The bioacoustic system processor 63 accomplishes the majority of the signal processing of the system. The bioacoustic system processor 63 is supplied with modified signals from the original multi-input signal generator 60 via the mixer and splitter 61 and the external signal processors, and is also supplied with an unmodified and unprocessed signal 64 from the multi-input signal generator 60 via the mixer and splitter 61 which is used for comparison purposes. The bioacoustic system processor 63 includes a field effect processor 70, which will be described in more detail later, as well as a plurality of internal signal processors. These internal signal processors may control and modify the amplitude, equalization and frequency, and operate to effect the dynamic range of the various signals output to the amplifiers 65. The internal signal processors may include expanders, filters and other similar signal modification devices.

After the signals supplied to the bioacoustic system processor 63 have been processed, they are output to the amplifiers 65. Preferably, there will be provided an amplifier for each similar group of transducers 66 used by the system, such as a first amplifier for audio speakers, a second amplifier for electromechanical vibrators and another amplifier for RF signals. The amplifiers 65 then provide amplified signals to the respective transducers 66 which convert the electrical signals into signals which effect the bioacoustical environment 67 within which the human user 68 is located. A direct feedback line from the processed outputs 72 is provided by the processed line signal 71. This processed line signal 71 is useful in the comparison process with the unprocessed signals 64 and the sensor signals 69.

The signals which are transmitted into the bioacoustical environment 67 and are not absorbed by the human user 68 are then received by a plurality of sensors 69 located within the bioacoustical environment 67. These sensors 69 convert each of the transduced signals from their respective medium into electrical signals which are then supplied back to the bioacoustic system processor 63. The sensors 69 will typically comprise one or more microphones, RF receivers and biostatic indicators. The received signals are then supplied to the bioacoustic system processor 63 to be analyzed and compared to the unprocessed signals 64 and the processed line signals 71 to obtain the acceptance/rejection profile of the human user 68 within the bioacoustical environment 67.

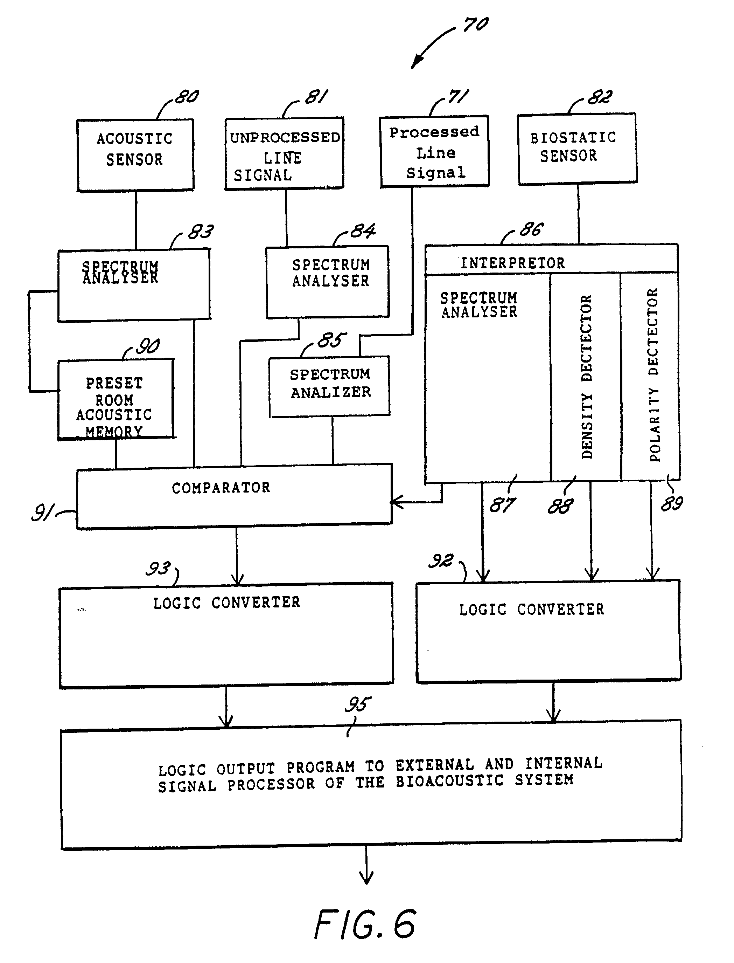

Turning now to Fig. 6, there is shown a general functional block diagram of the field effect processor 70 located in the bioacoustic system processor 63 of Fig. 5. As can be seen, there are four different signals being input to the field effect processor 70. A first signal is supplied from the acoustic sensors 80, such as microphones located within the bioacoustical environment, a second signal, the unprocessed line signal 81 is supplied from the multi-input signal generator, a third signal is supplied from the processed line signal 71 and a fourth signal is supplied from the biostatic sensors 82 located within the bioacoustical environment.

The frequency content and amplitude of the signals supplied from the acoustic sensors 80 are determined by a spectrum analyzer 83. Similarly, the frequency content and amplitude of the unprocessed line signals 81 and processed line signals 71 are determined by spectrum analyzers 84 and 85 respectively. During initialization of the system, the acoustic sensors 80 are used to determine the acoustic characteristics of the bioacoustical environment when the human user is not present. This allows the system to compensate for these pre-existing acoustic characteristics so as eliminate the effects of these characteristics to when determining the acoustical characteristics of the bioacoustical environment when the human user is present. These pre-existing acoustic characteristics are stored in the preset room acoustic memory 90, which is supplied from the spectrum analyzer 83. The signals from the preset room acoustic memory 90 are provided to the comparator 91 for use in the comparison process.

The system includes one or more biostatic sensors 82 or indicators which provide information relating to the biostatic state of the human user in the bioacoustic environment. A biostatic interpreter 86 properly distributes the signals from the biostatic sensor to a spectrum analyzer 87, density detector 88 and a polarity detector 89. Since the acoustic output to the bioacoustic environment may modulate the biostatic field, signals from the biostatic spectrum analyzer 87 are provided to the acoustic comparator 91 to compensate for this modulation. The signals from the biostatic spectrum analyzer 87, density detector 88 and polarity detector are then provided to a biostatic logic converter 92.

The acoustic comparator 91 operates to determine the acceptance/rejection profile of the human user in the bioacoustic environment, as well as simplifying the computation task of the acoustic logic converter 93. Since the comparator 91 determines the difference between the signals input to the bioacoustic environment and the signals received from the bioacoustic environment, the comparator 91 first subtracts the original unprocessed line signal 81 supplied by the spectrum analyzer 84 from the processed line signals 71 via the spectrum analyzer 85 and the acoustic signals 80 via the spectrum analyzer 83. Thus, this will produce two new signals which represent first, the difference between the processed line signals 71 and the unprocessed line signals 81, and a second signal which represents the difference between the acoustic signals 80 and the unprocessed line signals 81. The new processed line signal is then subtracted from the new acoustic signal to produce another new signal, the acceptance/rejection signal, which represents the difference between the signals output to the bioacoustic transducers and the signals received by the receivers from the bioacoustic environment.

By operation of the comparator 91, this acceptance/rejection signal represents the difference between the input and output signals, thereby representing the signals which the human user has absorbed. Since the speed in which changes in the biophysical state of the human user take place will typically be slight in comparison to the speed in which this acceptance/rejection signal is computed, for many frequencies its value will be marginal or zero. The frequencies which are changing most rapidly will be contain values of the greatest magnitude and will be used further in the processing of the signals. The frequencies which have experienced little or no change, i.e., very small acceptance/rejection signal values, typically will not be considered any further in the processing of the signals. This acceptance/rejection signal is then supplied to the acoustic logic converter 93 for further processing.

The logic converters 92 and 93 convert the various spectral information supplied to them into logic code which is read and analyzed by a programmed logic unit 95. The programmed logic unit 95 contains information regarding the predetermined desired biophysical state for the user. This predetermined information will typically contain the desired levels for the various frequencies being monitored by the system. The programmed logic unit 95 uses this predetermined information along with programmed routines programmed to output control logic signals to be input to the various signal processors of the bioacoustic system processor. Accordingly, the control signals output from the programmed logic unit 95 are supplied to the respective signals processor located within the bioacoustic system processor.

The programmed logic unit 95 of the field effect processor 70 is an important element of the present invention in that it functions as the main signal analysis and modification means required by the feedback system. The logic unit may be programmed in many ways and it is not the intent of the present invention to be limited to one such programmed configuration. In this regard, the function of the programmed logic unit will now be described in connection with a few examples in order to illustrate various advantageous ways in which the programmed logic unit may be programmed.

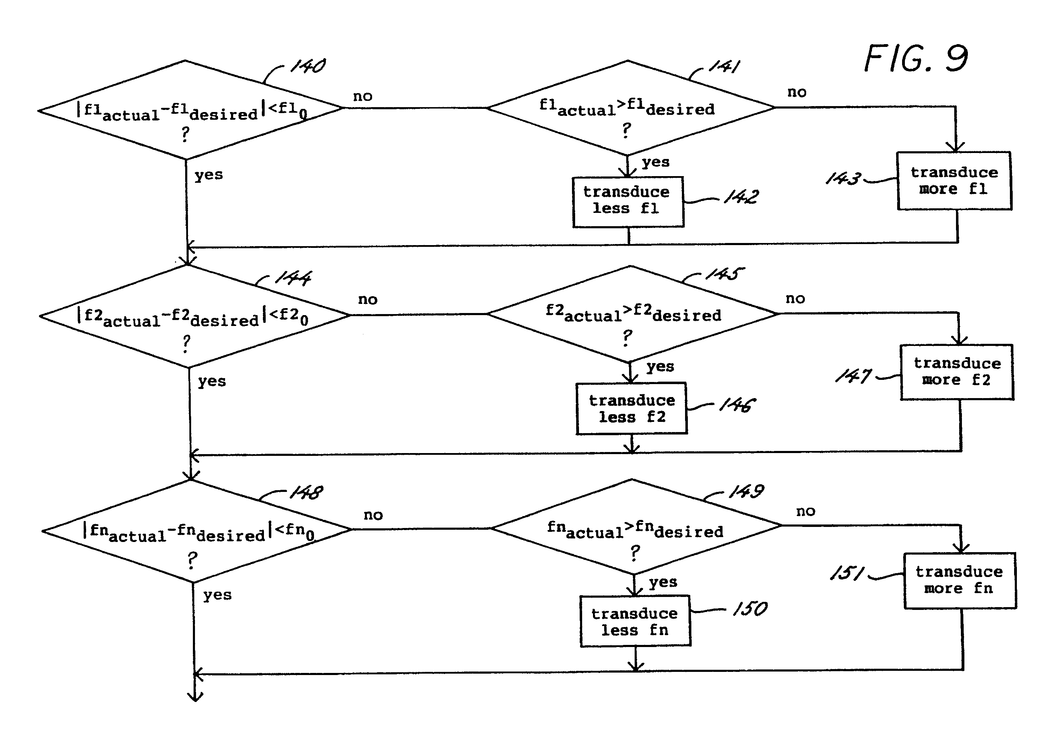

Turning now to Fig. 9, there is shown a flow diagram representing one example of operation of the programmed logic unit 95 of Fig. 6. As previously described, the logic converters 92 and 93 provide primarily spectral information relating to signals received from the sensors. Preferably, this spectral information will be in digital format to facilitate the processing procedure as by a digital computer system or microprocessor.

An example of the processing of the programmed logic unit as given in Fig. 9, is done with respect to a number of specific predetermined frequencies which may be of particular interest to the biophysical state of the user. The magnitude of these various frequencies as determined by the acceptance/rejectance profile for the user by the logic converters and is supplied to the programmed logic unit. A corresponding number of predetermined desired magnitudes of these frequencies is stored in the programmed logic unit for comparing the user's acceptance/rejectance profile at these particular frequencies to a predetermined desired acceptance/rejectance profile at these particular frequencies, respectively. Thus at a plurality of different frequencies at various points of the acceptance/rejectance profile, the programmed logic unit compares the actual spectral information related to the user to the predetermined desired spectral information stored within the unit.

In step 140 of the procedure, the magnitude of an actual frequency f1 is represented as $f1_{actual}$, while the magnitude of the corresponding predetermined frequency is represented as $f1_{desired}$. A predetermined magnitude threshold for the magnitude of the difference between the magnitude of the actual and desired frequencies, is represented as $f1_0$. A difference of the magnitude between these two desired and actual magnitude of these frequencies which is less than the threshold value will not be considered for further processing. On the other hand a difference which is not less than the threshold value will be used to modify the transduced signals in order to adjust the biophysical state of the user so as to bring the magnitude of the particular frequency to the predetermined desired magnitude.

In steps 140 through 143, the particular frequency f1 is examined and possibly used to alter the transduced signals of the system. Beginning with step 140 the predetermined desired magnitude of the frequency $f1_{desired}$ is subtracted from the actual magnitude of the frequency $f1_{actual}$ related to the acceptance/rejectance of the user at the particular frequency f1. The magnitude of this difference is then compared to the predetermined threshold value $f1_0$. If the difference is less than the threshold value $f1_0$ then this particular frequency f1 is not considered in the modification process and the procedure continues directly to step 144 for examining the next frequency f2.

If the difference in step 140 is not less than the predetermined threshold value $f1_0$, then the procedure continues to step 141. Step 141 determines whether the magnitude of the actual frequency is greater than the predetermined desired magnitude of the frequency $f1_{desired}$. If the actual magnitude is greater than the desired magnitude then the process continues to step 142 which signifies to the system to transduce less of the particular frequency f1.

If the magnitude of the actual frequency from the human user $f1_{actual}$ is not greater than the predetermined desired magnitude of the frequency $f1_{desired}$ then the procedure branches to step 143, signifying to transduce more of the frequency f1. After either step 142, which decreases the amount of transduced frequency f1, or step 143, which increases the amount of transduced frequency f1, the procedure continues to step 144 to examine the frequency f2.

Similarly in step 144, the predetermined desired magnitude of the frequency $f2_{desired}$ is subtracted from the actual magnitude of the frequency $f2_{actual}$ as determined from the sensors about the user. If the magnitude of this difference is less than the value of a predetermined desired magnitude for this frequency $f2_0$ then the procedure continues to step 148, otherwise this frequency f2 is modified in steps 145 through 147.

In step 145, if the magnitude of the actual frequency $f2_{actual}$ is greater than the predetermined desired magnitude $f2_{desired}$, then the procedure continues to step 146 to decrease the amount of frequency f2 which is transduced. Otherwise, the procedure branches to step 147 to increase the amount of frequency f2 transduced. Similarly, the procedure then continues to step 148 to examine another frequency fn to determine if it should be modified.

As can be seen in Fig. 9, any number of additional routines such as 140-143 or 144-147 may be used to examine a respective number of frequencies for possible modification. Thus, the procedure operates so as to determine the actual magnitudes of particular frequencies (such as $f1_{actual}$, $f2_{actual}$, etc.) which differ more than the value of a predetermined threshold (such as $f1_0$, $f2_0$, etc.). The frequency magnitudes which vary beyond the threshold value are used to modify the transduced frequencies. If the actual magnitude is greater than the predetermined desired magnitude, then less of the particular frequency being examined is transduced. If the actual magnitude is not greater than the predetermined desired magnitude, then more of the particular frequency being examined is transduced.

The system may be operated in a number of different ways in order to increase or decrease the amount of a particular frequency being transduced. The simplest manner in which a particular frequency may be modified would be to merely adjust the amplitude of the signal for the particular frequency. The transducers will respond by producing signals which reflect this change in amplitude.

Alternatively, the system may function so as to synthesize a new signal which is related to the particular frequency. For example, a new harmonically related frequency may be synthesized such as a signal which is twice the frequency, i.e. one octave higher, or a signal which is half the frequency, i.e. one octave lower, of the frequency of the particular signal. Similarly, a number of harmonically related frequencies may be transduced from several octaves below through several octaves above the particular frequency which is to be modified.

Alternatively, new mathematically related frequencies may be synthesized in order to modify the particular frequency. A new signal with a frequency which is a factor of Phi with respect to the particular frequency, may be synthesized. For example, it may be determined through the comparison process that a frequency of 438.847 hertz should be increased in amplitude. Since this frequency may already be set to a maximum level, a new related frequency may be synthesized by multiplying the particular frequency value by the value Phi, thereby synthesizing a new signal with a frequency of 710.054 hertz.

Therefore, to adjust to amount of a particular frequency transduced, the system may operate directly so as to increase or decrease the amplitude of the particular frequency as desired. Alternatively, the system may operate so as to make the adjustment indirectly by synthesizing or altering the amplitude of a harmonically or mathematically related frequency.

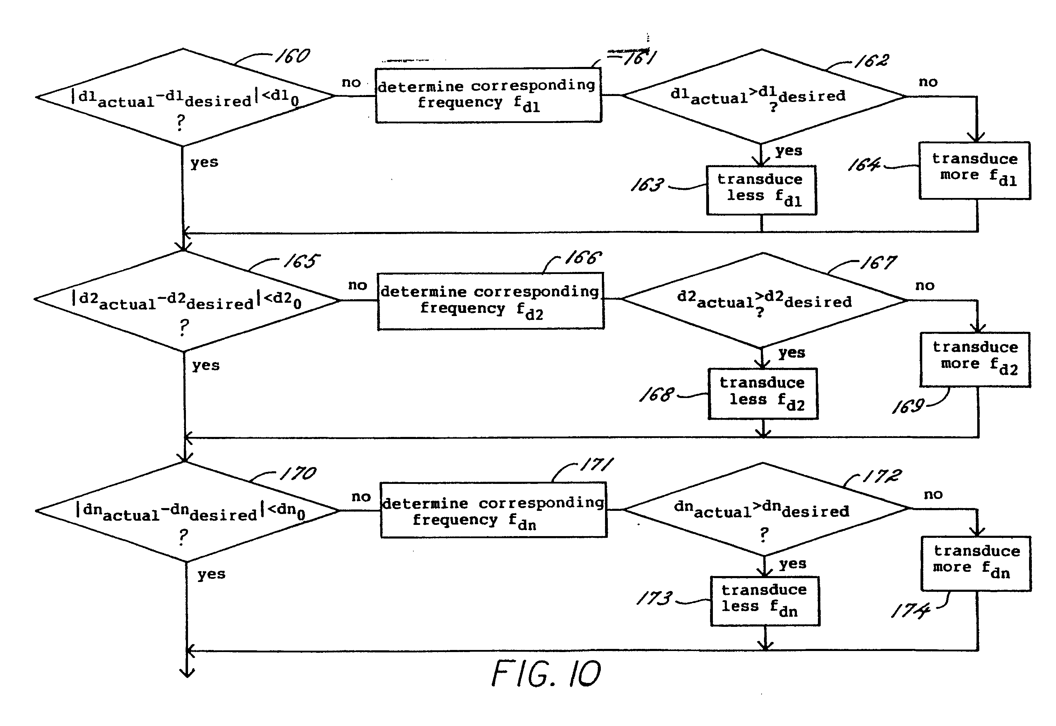

Turning now to Fig. 10, there is shown an example of a procedure in which the programmed logic unit may operate to utilize the density of the biostatic field of the human user to modify the transduced signals. Since the biostatic field is primarily utilized to monitor the biophysical state of the user, and biostatic signals are not transduced by the system, these signals must be analyzed and related to signals which are transduced in order to take part in the feedback aspect of the invention.

As previously described, it is evident that the density of the biostatic field of the user is related to the user's degree of relaxation. A higher density value indicates the user is more relaxed, whereas a lower density value indicates the user is more tense. Preferably, there will be several biostatic sensors located at certain areas of the human user. For example, one may be located near the neck of the user to monitor the relaxation of the head and neck muscles and surrounding area. In conjunction with Fig. 10, the density value of a first biostatic sensor is denoted d1, the density value of a second biostatic sensor is denoted d2 and the density value of an $n^{th}$ biostatic sensor is denoted dn. As is apparent from Fig. 10, a number of additional biostatic sensors may be included in the process by including additional procedure segments such as those of 160 through 164 and 165 through 169.

Similar to the operation with the various frequency magnitudes of Fig. 9, in Fig. 10 a predetermined desired density value for a particular biostatic sensor is subtracted from the actual density value of that sensor. With respect to the first biostatic sensor density value as used in step 160, the desired density value $d1_{desired}$ is subtracted from the actual density value $d1_{actual}$. If the magnitude of this difference is less than a predetermined threshold value $d1_0$ for this first sensor, then the procedure continues directly to step 165 without further considering the density value of this first sensor in the modification process.

Otherwise, if in step 160 the magnitude of the differences between the actual and desired density values is not less than the threshold value $d1_0$, then the procedure continues to step 161. Step 161 determines a corresponding frequency $f_{d1}$ which is effective for affecting the relax of the area of the body where this first biostatic sensor is located. For example, if the first sensor was located near the neck of the user, then the associated frequency would be one which is particularly effective at stimulating the neck area and muscles in that particular region of the body. Normally, this would typically be characterized as the resonant frequency for that region of the body.

After the corresponding frequency $f_{d1}$ is determined in step 161, the procedure continues to step 162 to determine how to alter this particular frequency. If in step 162 it is determined that the actual value of the density $d1_{actual}$ is greater than the desired value $d1_{desired}$, then the process continues to step 163 to transduce less of the corresponding frequency $f_{d1}$, thereby resulting in less of this particular stimulating frequency being transduce. Otherwise, it in step 162 the actual density value $d1_{actual}$ is not greater than the desired density value $d1_{desired}$, then the user is too tense in the area of the first sensor. In this case, the procedure branches to step 164, whereby more of the particular stimulating frequency $f_{d1}$ is transduced, thereby affecting the user with more of this frequency which will tend to relax him more in this area.

After the density value associated with the first biostatic sensor has been examined and possibly used for modifying the corresponding frequency, the procedure continues to step 165 to examine the density value d2 associated with a second biostatic sensor. Similarly, the actual density value $d2_{actual}$ is compared to the desired density value $d2_{desired}$, and if the magnitude of this difference is greater than a threshold value $d2_0$ then modification of the corresponding frequency $f_{d2}$ is accomplished similarly in steps 166 through 169. Otherwise, if the difference is less than the threshold value, then the procedure continues to step 170 where the density value of another biostatic sensor is examined.

One of the important objects of the present invention is to provide an environment conducive to the user's relaxation which will encourage and promote stress reduction. When a user initially uses the system he will probably not be relaxed, at least in certain areas of his body. For example, his chest muscles may be tensed, causing an increased heart rate and constricting his respiration. The nerve plexis area associated with the chest muscles may be specifically monitored by the system, since there is a particular unique resonant frequency associated with this nerve plexis area. When this nerve plexis. area is tense, the programmed logic unit will recognize this fact due to the high rejectance ratio, from the acceptance/rejectance profile, associated with this particular frequency as compared to a predetermined relaxed acceptance/rejectance profile. The programmed logic unit will utilize this fact, and in response, will increase the transduced signals for this particular frequency. The increase may take the form of increased amplitude or may be realized by adding a related octave for this particular frequency. For example in the audio signals, another tone may be added one octave higher or one octave lower, or several additional tones may be added several octaves higher or several octaves lower. Additionally, the volume of this particular signal may be increased. Similarly, a subaudio signal related to this particular frequency associated with the tense nerve plexis area may be added or increased in the audio vibrator or electromechanical vibrators attached to the table. The increased transduced signal for this particular frequency will stimulate this particular tensed nerve plexis area, thereby increasing the tendency for these muscles to become relaxed.

The acceptance/rejectance profile of the user may be utilized in combination with the information from the biostatic field sensors to provide a more conclusive determination of the user's biophysical condition. As will be explained in more detail later, the information from the biostatic field can be used to make a determination relating the user's particular emotional state. Using this emotional state in combination with the user's physical state, as derived from the biostatic field and the acceptance/rejectance profile, respectively, the programmed logic unit may much more accurately characterize the user's overall biophysical condition. For example, a user with a rather relaxed physical condition and a very dominant, yet transitional, emotional state may be suffering from depression. On the other hand, an individual with a tensed and energetic physical condition, as determined by the acceptance/rejectance profile, in combination with a very high and fluctuating emotional state, as determined by sensing the biostatic field, may be suffering from anxiety or paranoia. Accordingly, transduced signals should then be sent which stimulate the various areas of the body which will change the individual's present biophysical state to a more desirable biophysical state.

Thus, the programmed logic unit is capable of analyzing and determining the required changes which should be made to the user's biophysical environment. When interpreted properly, the user's biophysical signals are utilized to modify his own biophysical environment, which by the altered stimulation will affect his biophysical signals. Thus, a true and complete biophysical feedback system is realized.

According to an important aspect of a preferred embodiment of the present invention, the various frequencies used by the system are based on the universal constant Phi. The value of the Phi is approximately 1.6180339 and is used as the first value in order to create a bioacoustic scale. The modern musical scale was arbitrarily based on the note A being 440 hertz. The rest of the musical scale was generated by multiplying each frequency by the twelfth root of two, (approximately 1.059) in order to generate the frequency for the next highest note in the scale. For example, in order to generate the musical note A# according to the modern musical scale, the frequency of the note A which is 440, is multiplied by 1.059 to generate the value 465.96 which corresponds to the frequency of the note A#. As is well known, to generate a note which is one octave higher than a certain note, the frequency corresponding to that certain note is doubled.

When using the bioacoustic scale based on Phi, the note A is found to have a frequency of approximately 438.8473 hertz. The base note of the bioacoustic scale corresponds most closely to the modern musical note G# at a very low octave. When this base note is brought to the proper octave by repeatedly multiplying the frequency by 2, the value of G# is found to be approximately 414.2167. Then by multiplying this value by 1.059, the bioacoustic frequency of A is found to be 438.8473 hertz, rather than the modern frequency of 440 hertz.

The preferred frequency values for the bioacoustic scale are given in Table I. The top row of this table lists the various human organs and body parts and their associated notes on the bioacoustic scale. For example, the spinal cord (first column) is associated with the bioacoustic note C and its associated frequencies such as 7.54473 CPM, 61.1578 CPM, and 16.30876 CPS, etc. The associated frequency values correspond to "resonant" frequencies for the organ or body part. When a particular organ is stimulated by this resonant frequency, its effectiveness in the human body is increased. Thus, the organ will tend to react positively to this frequency. The most effective frequency range for the human organs is below 1000 hertz, due to the particular mass associated with the organs. For example, to stimulate the thyroid, frequencies should be transmitted to the human body, as by vibrations, such as 12.21777 hertz, 24.43553 hertz, 48.87106 hertz, etc.

In addition to the body organs, the bioacoustic scale may be used to stimulate a human being to produce a predetermined desired biophysical response. For example, transmitting the musical note B with the body pulsed to a heart rate of 57.72 cycles per minute and a phasing of stimulation from head to toe of 14.43 cycles per minute can induce spontaneous moods of creativity. Preferably, the values in the bioacoustic scale will be used throughout the system so as to create a biophysically pleasing environment. For example, in order to avoid the negative influence of a 60 hertz frequency of electromagnetic fields generated by modern electric power line voltages (i.e., a frequency which is not in the bioacoustic scale), a 58.12 hertz signal should be used to energize the system.

According to an important aspect of a preferred embodiment of the present invention, the frequency values of the bioacoustic scale are utilized by the field effect processor, in combination with the signals received by the sensors, to modify the signals sent to the transducers. The acceptance/rejectance profile determined primarily from the feedback of the audio signals, when used together with the information gathered by the biostatic sensors, the physical, emotional and psychological state of the human user is determined. In other words, substantially the entire biophysical condition of the human user is determined.

Since the particular biophysical condition of the user when initially using the system may not be desired, the system provides means for altering the human user's bioacoustical environment to create an atmosphere which is conducive to bringing the user to a more desirable biophysical condition. The means for altering the bioacoustical environment of the human user is accomplished by modifying the signals sent to the transducers which create the bioacoustical environment.

According to an important object of the invention, after a user has used the system and become familiar with it, he will be able to better control his biophysical condition by controlling his individual biophysical characteristics. Use of the system will increase the user's awareness of his biophysical characteristics. The majority of individuals have little or no awareness of even their most obvious biophysical characteristics. For example, most individuals are not aware of their respiration rate or know this characteristic may be related to relaxation. But after use of the system, an individual will gain an increased awareness to at least this basic biophysical characteristic. Additionally, an individual will understand this biophysical characteristic of respiration rate is directly related to the individual's relaxation. A more experienced user of the system will also be able to control a greater number of his biophysical characteristics by using the experience he has gained through the biophysical feedback loop utilized in the present invention. Thus, an individual's enhanced awareness and control of his biophysical characteristics will be realized.

## Claims

1. A system for enhancing a user's awareness to said user's biophysical characteristics, said system comprising in combination:
transducer means for producing a plurality of signals to affect the biophysical characteristics of said user;
sensor means for receiving a plurality of different biophysical signals from said user;
processing means for producing a difference signal by comparing the signals produced by said transducer means with the signals received from said sensor means; and
feedback means for using said difference signal to modify one or more predetermined signals and transmit said modified signal to said transducer means.

2. The system according to claim 1 further comprising synthesizing means for synthesizing signals in response to said difference signal, wherein said synthesized signals are provided to said transducer means.

3. The system according to claim 1 wherein said sensor means includes one or more biostatic sensors for receiving signals related to the biostatic field of said user.

4. The system according to claim 3 wherein said sensor means determines the frequency, polarity and density of the signals received by said biostatic sensors related to the biostatic field of said user.

5. The system according to claim 3 wherein said biostatic sensors are disposed so as to receive biostatic signals related to the difference between the biostatic field on the right side of said user and the biostatic field on the left side of said user.

6. The system according to claim 3 wherein said biostatic sensors are disposed so as to receive biostatic signals related to the difference of the biostatic field of an upper portion of said user and the biostatic field of a lower portion of said user.

7. The system according to claim 1 wherein said transducer means includes a multichannel speaker system for producing audio signals.

8. The system according to claim 7 wherein said multichannel speaker system comprises

a right speaker portion for producing audio signals on one side of said user,

a left speaker portion for producing audio signals on the other side of said user, and

a differential speaker portion for producing signals related to the relative difference between the signals produced by said right speaker portion and the signals produced by said left speaker portion.

9. The system according to claim 1 wherein said transducer means includes a multichannel signal generator, said signal generator comprising

a first channel for generating a first set of signals,

a second channel for generating a second set of signals,

wherein said first set of signals and said second set of signals are used to produce a third unique differential set of signals related to the relative difference between said first set of signals and said second set of signals.

10. The system according to claim 9 wherein said multichannel signal generator further comprises

a plurality of additional channels for generating a respective plurality of additional sets of signals,

wherein said first set of signals and said second set of signals and said plurality of additional sets of signals are used to produce a plurality of unique differential sets of signals related to the relative difference between each combination of two sets of signals.

11. The system according to claim 1 wherein said user lies on a generally horizontally disposed table suspended within a framework.

12. The system according to claim 11 wherein the signals produced by said transducer means include subaudio signals produced by one or more electromechanical transducers transmitting said subaudio signals to said table.

13. The system according to claim 12 wherein said electromechanical transducers are operated in two groups, wherein a first of said two groups is disposed on one half of said table and a second of said two groups is disposed on the other half of said table.

14. The system according to claim 13 wherein said two groups of electromechanical transducers are alternately energized at substantially a biophysical harmonic rate.

15. A method for enhancing a user's awareness to said user's biophysical characteristics, said method comprising the steps of:

generating a number of different biophysical signals and supplying said generated signals to said user;

receiving a number of different biophysical signals emanating from said user relating to said user's response to the signals supplied to said user;

comparing said generated signals to said received signals to produce a difference signal; and

using said difference signals to modify one or more predetermined signals, generating biophysical signals from said modified signal and producing said modified biophysical signal to said user.

16. The method according to claim 15 further comprising the step of synthesizing signals in response to said difference signal.

17. The method according to claim 15 wherein said received signals include signals related to the biostatic field of said user which are received from one or more biostatic sensors.

18. The method according to claim 17 wherein the polarity, density and frequency of said received signals is determined.

19. The method according to claim 17 wherein said biostatic sensors are disposed so as to receive biostatic signals related to the difference between the biostatic field on the right side of said user and the biostatic field on the left side of said user.

20. The method according to claim 17 wherein said biostatic sensors are disposed so as to receive biostatic signals related to the difference of the biostatic field of an upper portion of said user and the biostatic field of a lower portion of said user.

21. The method according to claim 15 wherein said generated signals include audio signals produced by a multichannel speaker system.

22. The method according to claim 21 wherein said multichannel speaker system comprises

a right speaker portion for producing audio signals on one side of said user,

a left speaker portion for producing audi signals on the other side of said user, and

a differential speaker portion for producing a signal related to the relative difference between the signals produced by the right speaker portion and the signals produced by the left speaker portion.

23. The method according to claim 15 wherein said generated signals are produced by a multichannel signal generator, said signal generator comprising

a first channel for generating a first set of signals,

a second channel for generating a second set of signals,

wherein said first set of signals and said second set of signals are used to produce a third unique differential set of signals related to the relative difference between said first set of signals and said second set of signals.

24. The method according to claim 23 wherein said multichannel signal generator further comprises

a plurality of additional channels for generating a respective plurality of additional sets of signals,

wherein said first set of signals and said second set of signals and said plurality of additional sets of signals are used to produce a plurality of unique differential sets of signals related to the relative difference between each combination of two sets of signals.

25. The method according to claim 15 wherein said received signals are received at least in part by one or more audio microphones.

26. The method according to claim 15 wherein said user lies on a generally horizontally disposed table suspended with a framework.

27. The method according to claim 26 wherein said generated signals include subaudio signals produced by one or more electromechanical transducers transmitting said subaudio signals to said table.

28. The method according to claim 27 wherein said electromechanical transducers are operated in two groups, wherein a first of said two groups is disposed on one half of said table and a second of said two groups is disposed on the other half of said table.

29. The method according to claim 28 wherein said two groups of electromechanical transducers are alternately energized at substantially a biophysical harmonic rate.

FIG. 1

FIG. 2

FIG. 3

FIG. 4

FIG. 5

FIG. 6

FIG. 7A

FIG. 7B

FIG. 7C

FIG. 7D

FIG. 8A

FIG. 8B

FIG. 9

$|f1_{actual} - f1_{desired}| < f1_0$ ? — 140

no → $f1_{actual} > f1_{desired}$ ? — 141

no → transduce more f1 — 143

yes → transduce less f1 — 142

yes ↓

$|f2_{actual} - f2_{desired}| < f2_0$ ? — 144

no → $f2_{actual} > f2_{desired}$ ? — 145

no → transduce more f2 — 147

yes → transduce less f2 — 146

yes ↓

$|fn_{actual} - fn_{desired}| < fn_0$ ? — 148

no → $fn_{actual} > fn_{desired}$ ? — 149

no → transduce more fn — 151

yes → transduce less fn — 150

yes ↓

EP 0 422 253 A1

FIG. 10

EP 0 422 253 A1

## BIOACOUSTIC SCALE BASED ON PHI (~1.618)

### TABLE I

|  | SPINAL | GONADS | PANCREAS |  | ADRENALS | THYMUS |  |
|---|---|---|---|---|---|---|---|
|  | RED | RED/ORANG | ORANGE | ORANG/YEL | YELLOW | GREEN | GREEN/BLU |
|  | C | C# | D | D# | E | F | F# |
| CPM | 7.54473 | 8.09931 | 8.58092 | 9.09117 | 9.63176 | 10.2045 | 10.8113 |
| | 15.2395 | 16.1986 | 17.1618 | 18.1823 | 19.2635 | 20.4090 | 21.6226 |
| | 30.5789 | 32.3972 | 34.3237 | 36.3647 | 38.5270 | 40.8180 | 43.2451 |
| | 61.1578 | 64.7944 | 68.6473 | 72.7293 | 77.0540 | 81.6359 | 86.4926 |
| CPS (HERTZ) | 2.0385 | 2.1598 | 2.288 | 2.424 | 2.568 | 2.721 | 2.883 |
| | 4.077 | 4.3196 | 4.576 | 4.848 | 5.136 | 5.442 | 5.766 |
| | 8.154380 | 8.639265 | 9.152982 | 9.697247 | 10.27388 | 10.88479 | 11.53204 |
| | 16.30876 | 17.27853 | 18.30596 | 19.39449 | 20.54775 | 21.76958 | 23.06407 |
| | 32.51752 | 34.55706 | 36.61193 | 38.78899 | 41.09550 | 43.55917 | 46.12814 |
| | 65.23504 | 69.11412 | 73.22386 | 77.57798 | 82.19100 | 87.07833 | 92.25628 |
| | 130.4701 | 138.2282 | 146.4477 | 155.1560 | 164.3820 | 174.1567 | 184.5126 |
| | 260.9402 | 276.4565 | 292.8954 | 310.3119 | 328.7640 | 348.3133 | 369.0251 |
| | 521.3803 | 552.9130 | 585.7909 | 620.6238 | 657.5280 | 696.6267 | 738.0593 |
| | 1043.761 | 1105.826 | 1171.582 | 1241.248 | 1315.056 | 1393.253 | 1476.101 |
| | 2087.521 | 2211.652 | 2343.164 | 2482.495 | 2630.112 | 2786.507 | 2952.201 |
| | 4175.043 | 4423.304 | 4686.327 | 4964.991 | 5260.224 | 5573.013 | 5904.402 |
| | 8350.085 | 8846.507 | 9372.564 | 9929.981 | 10520.45 | 11146.03 | 11808.80 |

| THYROID |  | PITUITARY |  | PINEAL |  |
|---|---|---|---|---|---|
| BLUE | BLU/INDIGO | INDIGO | INDIGO/VIO | VIOLET | ← ORGANS / ← COLOR |
| G | G# | A | A# | B | ← MUSICAL NOTES |
| 11.4542 | 12.1353 | 12.8569 | 13.6214 | 14.4313 | } RESPIRATION |
| 22.9083 | 24.2705 | 25.7137 | 27.2427 | 28.8627 | |
| 45.8166 | 48.5410 | 51.4274 | 54.4855 | 57.7253 | } HEART RATE |
| 91.6332 | 97.0820 | 102.855 | 108.971 | 115.451 | |
| 3.054 | 3.236 | 3.428 | 3.632 | 3.848 | |
| 6.108 | 6.472 | 6.856 | 7.264 | 7.696 | } BRAIN WAVES |
| 12.21777 | 12.94427 | 13.71398 | 14.52945 | 15.39342 | |
| 24.43553 | 25.88854 | 27.42796 | 29.05891 | 30.78684 | |
| 48.87106 | 51.77709 | 54.85591 | 58.11782 | 61.57368 | |
| 97.74213 | 103.5542 | 109.7118 | 116.2356 | 123.1474 | |
| 195.4843 | 207.1084 | 219.4237 | 232.4713 | 246.2947 | |
| 390.9685 | 414.2167 | 438.8473 | 464.9425 | 492.5894 | |
| 781.9370 | 828.4334 | 877.6946 | 929.8851 | 985.1789 | } AUDIO AND MUSIC |
| 1563.874 | 1656.867 | 1755.389 | 1859.770 | 1970.358 | |
| 3127.748 | 3313.734 | 3510.778 | 3719.540 | 3940.716 | |
| 6255.496 | 6627.467 | 7021.557 | 7439.080 | 7881.431 | |
| 12510.99 | 13254.93 | 14043.11 | 14878.16 | 15762.86 | |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-3 942 516 (T.W. GLYNN)<br>* Column 3, lines 10-54 * | 1,15 | A 61 M 21/00<br>A 61 B 5/00 |
| A | US-A-3 648 686 (B.R. PAYNE)<br>* Column 2, lines 8-20; column 4, lines 22-25 * | 1,15 | |

TECHNICAL FIELDS
SEARCHED (Int. Cl.5)

A 61 M
A 61 B

The present search report has been drawn up for all claims

| Place of search | Date of completion of the search | Examiner |
|---|---|---|
| THE HAGUE | 22-05-1990 | GERARD B.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)